# EUROPEAN PATENT SPECIFICATION

(11) **EP 2 110 380 B1**
(45) Date of publication and mention of the grant of the patent: **10.11.2010**
(21) Application number: 09157585.2
(22) Date of filing: 08.04.2009
(51) Int. Cl.: C07K 1/04, C07K 1/06, C07K 1/107, C07K 5/06, C07C 227/18, C07D 307/81

(54) **Alpha-N-methylation of amino acids**
Alpha-N-Methylierung von Aminosäuren
Alpha-N-méthylation d'acides aminés

(30) Priority: 18.04.2008 US 46143 P
(43) Date of publication of application: 21.10.2009
(73) Proprietor: F. Hoffmann-La Roche AG, 4070 Basel (CH)
(72) Inventor: Moss, Jason, A., San Diego, CA 92122 (US); Swistok, Joseph, Nutley, NJ 07110 (US)
(74) Representative: Küng, Peter

(56) References cited:
- PLESS J: "53. EIGENSCHAFTEN UND VERWENDBARKEIT DES 10,11-DIHYDRO-5H- DIBENZO not A,D 3/4 CYCLOHEPTEN-5-YL-RESTS (=5-DIBENZOSUBERYL-RESTS) ALS NEUE SCHUTZGRUPPE FUER AMINE, AMINOSAEUREN, ALKOHOLE, THIOLE UND CARBONSAEUREN" HELVETICA CHIMICA ACTA, VERLAG HELVETICA CHIMICA ACTA. BASEL, CH, vol. 59, no. 2, 1 January 1976 (1976-01-01), pages 499-512, XP000929226 ISSN: 0018-019X
- GREEN T W ET AL: "Protective Groups in Organic Synthesis, Special -NH Protective Groups, Dbs-NR2" 1 January 1991 (1991-01-01), PROTECTIVE GROUPS IN ORGANIC SYNTHESIS, NEW YORK, WILEY & SONS, US, PAGE 583 , XP002533152 *section "N-5-dibenzosuberylamine (Dbs-NR2)"*
- KALJUSTE K ET AL: "NEW METHOD FOR THE SYNTHESIS OF N-METHYL AMINO ACIDS CONTAINING PEPTIDES BY REDUCTIVE METHYLATION OF AMINO GROUPS ON THE SOLID PHASE" INTERNATIONAL JOURNAL OF PEPTIDE AND PROTEIN RESEARCH, MUNKSGAARD, COPENHAGEN, DK, vol. 42, no. 2, 1 August 1993 (1993-08-01), pages 118-124, XP000464145 ISSN: 0367-8377
- BIRON ERIC ET AL: "Optimized selective N-methylation of peptides on solid support." JOURNAL OF PEPTIDE SCIENCE : AN OFFICIAL PUBLICATION OF THE EUROPEAN PEPTIDE SOCIETY MAR 2006, vol. 12, no. 3, March 2006 (2006-03), pages 213-219, XP002533150 ISSN: 1075-2617
- BIRON ERIC ET AL: "Convenient synthesis of N-methylamino acids compatible with Fmoc solid-phase peptide synthesis." THE JOURNAL OF ORGANIC CHEMISTRY 24 JUN 2005, vol. 70, no. 13, 24 June 2005 (2005-06-24), pages 5183-5189, XP002533151 ISSN: 0022-3263

## Description

The present invention relates to synthesis of ^{α}*N*-methylated amino acids.

In peptide synthesis, amide bond ^{α}*N*-methylation often serves to abrogate proteolytic susceptibility, enhancing the stability of the peptide with minimal structural perturbation. Methylation of amino acids dates back to the work of Emil Fischer, who was the first to achieve monomethylation of α -amino acids (Fischer, E.; Lipschitz, W. Chem. Ber. 1915, 48, 360.) Fischer's three-step synthetic route comprised the following three key steps: transient protection of the primary α-amino group to leave a single N-H group, ^{α}*N*-methylation to replace the N-H with an N-CH₃ group, and deprotection of the transient protecting group to liberate the now-secondary, ^{α}*N*-methyl amino acid. Notwithstanding differences in the chemical minutiae, the same three key steps are still used in many ^{α}*N*-methylation chemistries employed today, some 100 years since Fischer's seminal work.

Analyzed in detail, Fischer's original chemistry was problematic for two reasons. First, his use of a toluenesulfonamide (tosylamide) protecting group in the first step as transient protection mandates an exceedingly harsh deprotection chemistry in the third step - conc. HCl at reflux - which is incompatible with amide bonds and in fact with many proteinogenic side-chains. Second, the methylation chemistry in the second step occurs under strongly alkylating and racemization-promoting conditions. For these reasons, most synthetic effects since then have been focused on the development of milder methodologies for this same three-step reaction sequence.

The primary improvement in ^{α}*N*-methylation chemistry was reported by Quitt et al., in which the Leukart reaction was used for the methylation of ^{α}*N*-benzyl amino acids (Quitt, P. In Proceedings of the 5th European Peptide Symposium Oxford, UK, 1963, p 165-169).

The mildness and chemoselectivity of this reaction - both critical for functional group tolerance and, by corollary, general applicability - allowed access to stereochemically-pure ^{α}*N-*methyl amino acids with a range (though not all) of the proteinogenic functional groups, as later elaborated by Ebata et al. (Ebata, M.; Takahashi, Y.; Otsuka, H. Bull. Chem. Soc. Jpn. 1966, 39, 2535). As these reactions achieve Fischer's third step - N-deprotection - via catalytic hydrogenation, catalyst poisoning by sulfur-containing amino acids, reduction of Trp indoles, and insolubility of the amino acids in hydrogenation reactions are all problems which manifested in generally low yields. Nonetheless, this chemistry set the benchmark for subsequent syntheses of stereochemically-pure ^{α}*N*-methyl amino acids.

Since the demonstrated efficacy of the Leukart reaction for ^{α}*N*-methylation, a number of other methods for this transformation have been reported over the subsequent years, with varying degrees of complexity. These have been reviewed in detail elsewhere, but were mostly innovations which allowed access to specific structures, such as natural product synthons, and were not intended as generally-applicable methodologies for ^{α}*N*-methylation (Aurelio, L.; Brownlee, R. T. C.; Hughes, A. B. Chem. Rev. 2004, 104, 5823-5846; Sagan, S.; Karoyan, P.; Lequin, O.; Chassaing, G.; Lavielle, S. Curr. Med. Chem. 2004, 11, 2799-2822). One notable class of chemistries centered around 5-oxazolidinones of ^{α}*N*-carbamoyl- or acylamino acids, which were in general prepared by cyclodehydration from a formaldehyde source and then reduced to yield the ^{α}*N-*protected, ^{α}*N*-methyl amino acids (Reddy, G. V.; Rao, G. V.; Iyengar, D. S. Tetrahedron Lett. 1998, 39, 1985-1986; Freidinger, R. M.; Hinkle, J. S.; Perlow, D. S.; Arison, B. H. J. Org. Chem. 1983, 48, 77). Another noteworthy (and elegant) chemistry involves the Aza-Diels Alder reaction, wherein a methyliminium intermediate is trapped by cycloaddition with cyclopentadiene, followed by acid-catalyzed cycloreversion and silane reduction to yield the ^{α}*N*-methylamino acid (Grieco, P. A.; Bahsas, A. J. Org. Chem. 1987, 52, 5746-5749).

All of the aforementioned chemistries are essentially inapplicable to methylation on the solid-phase for two principal reasons. First, most employ catalytic hydrogenation, which has long been recognized to be incompatible with solid-phase synthesis due to the virtual impenetrability of the solid support to catalyst particles employed in these reactions. Second, for the chemistries that employ transient ^{α}*N*-protecting groups not removable by catalytic hydrogenation, the final deprotection step is accomplished by harsh acids, which are incompatible with many protecting groups and peptide-resin linkages used in modem SPPS (solid phase peptide synthesis).

It was not until Kaljuste and Undén's innovation of a novel three-step chemistry that reductive methylation could be employed on the solid phase for peptide synthesis according to the Boc/Bzl strategy (Kaljuste, K.; Undén, A. Int. J. Pept. Prot. Res. 1993, 42, 118-124).

In this chemistry, the 4,4'-dimethoxydiphenylmethyl chloride (Dod-Cl) is used as the alkylating agent for the ^{α}*N*-deprotected peptide resin. In the second step, the now-secondary ^{α}*N-*terminus is reductively methylated using formaldehyde and NaCNBH₃ as the reducing agent. The final deprotection step is then accomplished with 1:1 TFA:CH₂Cl₂ on the solid phase, liberating the Dod cation and the newly ^{α}*N*-methyl terminal amino acid residue. The benefits of this chemistry stem from its mildness - the absence of strong base, SN2 alkylation, heterogeneous catalysis, and heat - and all render it completely appropriate for on-resin ^{α}*N*-methylation in the context of Boc/Bzl SPPS.

However, this chemistry requires a strong acid to be used in the final deprotection step; the deprotection of the ^{α}*N* -Dod terminus mandates prolonged (1 hr) exposure to high concentrations of TFA (50% v/v in CH₂Cl₂). Thus, this chemistry is fundamentally incompatible with modem Fmoc/tBu SPPS, wherein such high concentrations of acid would effectively remove side-chain protecting groups and/or cleave the peptide-resin linkage.

The first methylation chemistry compatible with on-resin methylation in conjunction with Fmoc/tBu SPPS was Fukuyama's nitrobenzenesulfonamide chemistry (Fukuyama, T.; Jow, C. K.; Cheung, M. Tetrahedron Lett. 1995, 36, 6373-6374, Miller, S. C.; Scanlan, T. S. J. Am. Chem. Soc. 1997, 119, 2301-2302, Yang, L. H.; Chiu, K. L. Tetrahedron Lett. 1997, 38, 7307-7310).

In this chemistry, the ^{α}*N* -terminus is sulfonylated with 2- or 2,4-dinitrobenzenesulfonyl chloride. In the next step, the sulfonamide nitrogen is alkylated - under SN2 or Mitsunobu conditions - to yield the N-methyl sulfonamide. This sulfonamide is then deprotected on the solid phase by a thiol nucleophile such as thiophenol, leaving the now secondary, ^{α}*N*-methyl terminus. This chemistry has the benefit of being completely orthogonal, in theory, to the side-chain protecting groups and peptide-resin anchorages commonly employed in Fmoc/tBu SPPS, and has been used for the preparation of selected single ^{α}*N*-methyl amino acids on the solid phase, as well as short peptides (Lin, X. D.; Dorr, H.; Nuss, J. M. Tetrahedron Lett. 2000, 41, 3309-3313, Biron, E.; Chatterjee, J.; Kessler, H. Journal of Peptide Science 2006, 12, 213-219, Biron, E.; Kessler, H. J. Org. Chem. 2005, 70, 5183-5189).

However, even in the first publication on this chemistry, it was shown to be either sparingly or completely incompatible with amino acids bearing nucleophilic side-chains, notably Met and Arg(Pbf). These chemoselectivity problems have been subsequently reported elsewhere by Rivier et al., who opted instead to use Undén's chemistry (in a Boc/Bzl synthesis) owing to its greater side-chain compatibility (Erchegyi, J.; Hoeger, C. A.; Low, W.; Hoyer, D.; Waser, B.; Eltzschinger, V.; Schaer, J. C.; Cescato, R.; Reubi, J. C.; Rivier, J. E. J. Med. Chem. 2005, 48, 507-514). The incompatibility of Fukuyama's chemistry with Arg(Pbf) likely stems from the similar pKa of the nitrobenzenesulfonamide and the Pbf sulfonamide, whereby the Pbf-protected guanidine is alkylated as well as the intended N-terminal nitrobenzenesulfonamide.

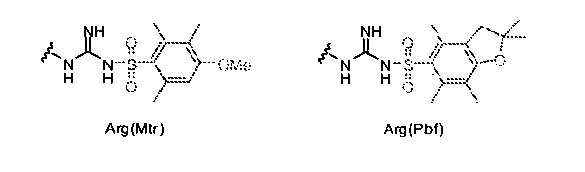

In the years following the application of the Fukuyama chemistry to SPPS, Laplante and Hall reported a variation of the Matteson rearrangement for on-resin methylation (Laplante, C.; Hall, D. G. Org. Lett. 2001, 3, 1487-1490). However, this chemistry is operationally complex and employs a potent oxidant treatment; as a result it is incompatible with oxidizable side-chains such as Met, Cys(Trt), and Trp, and is therefore of limited utility in peptide chemistry.

In Fmoc SPPS, the Arg side chain is commonly masked with the 2,2,4,6,7-pentamethyldihydrobenzofuran-5-sulfonamide (Pbf) group, which can be smoothly deprotected under relatively mild global deprotection conditions without detriment to other proteinogenic functionalities. In the synthesis of peptides bearing N-methyl Arg residues, Fmoc-MeArg(Mtr)-OH may be used (incorporating the more acid-stable 4-methoxy-2,3,6-trimethylbenzene*-sulfamide protecting group) as a synthon to avoid the step of on-resin ^{α}*N*-methylation entirely. However, Arg(Mtr) is not a viable alternative to Arg(Pbf). This is because the global deprotection step in the presence of Arg(Mtr) is attended by a number of serious side reactions which result in unacceptably low yields and complex chromatographic purification. These problems can be ascribed to the use of the Mtr protecting group on ^{α}*N*-methylated Arg (MeArg) residues because this protecting group is highly acid-stable, requiring harsh, long-term acid treatment during the global deprotection step for its removal from the Arg side chain. Moreover, it is occasionally only partially removed, and the thus-removed sulfonyl cation can covalently modify other residues such as Trp. Thus, Arg(Mtr) is not an effective alternative to the more acid labile Arg(Pbf) in a peptide build scale up procedure requiring incorporation of an ^{α}*N*-methylated Arg.

Therefore, there exists a need for ^{α}*N*-methylation chemistry suitable for on-resin methylation compatible with Fmoc/tBu SPPS and with amino acids bearing protected or unprotected nucleophilic side-chains, notably Arg(Pbf), Met, Cys, and Trp.

### SUMMARY OF THE INVENTION

The application provides a

method for ^{α}*N*-methylating of an amino acid or a peptide on a solid phase resin comprising the steps of:
a) contacting the amino acid or peptide with 5-chlorodibenzosuberane (Dbs-Cl),
b) contacting the product of step a) with formaldehyde or a protected formaldehyde equivalent,
c) contacting the product of step b) with a reducing agent; and
d) contacting the product of step c) with 5% TFA.

In certain embodiments of the above method, the reducing agent is NaCNBH₃ or NaBH(OAc)₃.

In certain embodiments of the above methods, the amino acid further comprises a side-chain protecting group.

In certain embodiments of the above methods, the amino acid is Arg(Pbf).

In certain embodiments of the above methods, the amino acid is methionine.

In certain embodiments of the above methods, the amino acid is tryptophan.

In certain embodiments of the above methods, the amino acid is cysteine.

The application provides a method for ^{α}*N*-methylating Arg(Pbf) on a solid phase resin comprising the steps of:
a) Fmoc deprotecting Fmoc-protected Arg(Pbf);
b) contacting the product of step a) with Dbs-Cl;
c) contacting the product of step b) with formaldehyde or a protected formaldehyde equivalent;
d) contacting the product of step c) with a reducing agent; and
e) contacting the product of step d) with 5% TFA.

The application provides the above method, wherein the reducing agent is NaCNBH₃ or NaBH(OAc)₃.

### Definitions

The phrase "a" or "an" entity as used herein refers to one or more of that entity; for example, a compound refers to one or more compounds or at least one compound. As such, the terms "a" (or "an"), "one or more", and "at least one" can be used interchangeably herein.

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components.

As used herein, unless specifically indicated otherwise, the word "or" is used in the "inclusive" sense of "and/or" and not the "exclusive" sense of "either/or".

The term "independently" is used herein to indicate that a variable is applied in any one instance without regard to the presence or absence of a variable having that same or a different definition within the same compound. Thus, in a compound in which R appears twice and is defined as "independently carbon or nitrogen", both R's can be carbon, both R's can be nitrogen, or one R can be carbon and the other nitrogen.

When any variable occurs more than one time in any moiety or formula depicting and describing compounds employed or claimed in the present invention, its definition on each occurrence is independent of its definition at every other occurrence. Also, combinations of substituents and/or variables are permissible only if such compounds result in stable compounds.

The term "optional" or "optionally" as used herein means that a subsequently described event or circumstance may, but need not, occur, and that the description includes instances where the event or circumstance occurs and instances in which it does not. For example, "optionally substituted" means that the optionally substituted moiety may incorporate a hydrogen or a substituent.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

As used herein, "amino acid" refers to a group represented by R'-NH-CH(R)-C(O)-R', wherein each R' is independently hydrogen, an aliphatic group, a substituted aliphatic group, an aromatic group, another amino acid, a peptide or a substituted aromatic group. Examples of amino acids include, alanine, valine, leucine, isoleucine, aspartic acid, glutamic acid, serine, threonine, glutamine, asparagine, arginine, lysine, ornithine, proline, hydroxyproline, phenylalanine, tyrosine, tryptophan, cysteine, methionine and histidine. R can be hydrogen or a protected or unprotected side-chain of a naturally-occurring amino acid.

An used herein, naturally occurring "amino acid side-chains" include methyl (alanine), iso-propyl (valine), sec-butyl (isoleucine), -CH₂CH(-CH₃)₂ (leucine), benzyl (phenylalanine), p-hydroxybenzyl (tyrosine), -CH₂-OH (serine), -CHOHCH₃ (threonine), -CH₂-3-indoyl (tryptophan), -CH₂COOH (aspartic acid), -CH₂CH₂COOH (glutamic acid), -CH₂C(O)NH₂ (asparagine), -CH₂CH₂C(O)NH₂ (glutamine), -CH₂SH, (cysteine), -CH₂CH₂SCH₃ (methionine), -[(CH₂)]₄NH₂ (lysine), -[(CH₂)]₃NH₂ (ornithine), -[(CH)₂]₄NHC(=NH)NH₂ (arginine) and -CH₂-3-imidazoyl (histidine).

Side-chains of amino acids comprising a heteroatom-containing functional group, e.g., an alcohol (serine, tyrosine, hydroxyproline and threonine), an amine (lysine, ornithine, histidine and arginine), may require a protecting group to facilitate reactions discussed herein. When the heteroatom-containing functional group is modified to include a protecting group, the side-chain is referred to as the "protected side-chain" of an amino acid. When the heteroatom-containing functional group is not modified to include a protecting group, the side-chain is referred to as the "unprotected side-chain" of an amino acid. Protecting groups are commonly used in peptide synthesis and these are known to, and often used by, the ordinary practitioner. For example, many suitable protecting groups, and methods for the preparation of protected amino acids, can be found in Green et al., Protecting Groups In Organic Synthesis, Third Edition, John Wiley & Sons, Inc. New York, 1999.

As used herein, the term "protecting group" or "transient protecting group" refers to a chemical group that is reacted with, and bound to, a functional group in a molecule to prevent the functional group from participating in subsequent reactions of the molecule but which group can subsequently be removed to thereby regenerate the unprotected functional group. Additional reference is made to: Oxford Dictionary of Biochemistry and Molecular Biology, Oxford University Press, Oxford, 1997 as evidence that "protecting group" is a term well-established in field of organic chemistry. Some common amine protecting groups include Aloc, Cbz, Dde, Fmoc, Trt and t-Boc.

As used herein, the phrase "amino acid or side-chain protected derivative" means a natural or unnatural amino acid or a derivative thereof that further comprises a protecting group on its side-chain. For example, "amino acid or side-chain protected derivative" includes both Arg and Arg(Pbf).

The term "protected formaldehyde equivalent" includes 1,3,5-trioxane, 1,3-dioxane, 1,3-dioxolane, formadehyde dimethyl acetal (or higher order alkyl acetals), paraformaldehyde, and DMSO.

As used herein "support", "solid support" or "solid phase" refers to any solid phase material upon which an amino acid or peptide is synthesized, attached, ligated or otherwise immobilized. Support encompasses terms such as "resin", "solid phase", "surface" and "solid support". A support may be composed of organic polymers such as polystyrene, polyethylene, polypropylene, polyfluoroethylene, polyethyleneoxy, and polyacrylamide, as well as co-polymers and grafts thereof. A support may also be inorganic, such as glass, silica, controlled-pore-glass (CPG), or reverse-phase silica. The configuration of a support may be in the form of beads, spheres, particles, granules, a gel, or a surface. Surfaces may be planar, substantially planar, or non-planar. Supports may be porous or non-porous, and may have swelling or non-swelling characteristics. A support may be configured in the form of a well, depression or other container, vessel, feature or location.

The term "resin" or "solid phase resin" includes poly(styrene-co-divinylbenzene) (PS-DVB) resins, poly(ethylene glycol) (PEG) based resins, 'hybrid' resins comprised of both PEG and polystyrene components, and silica- and methacrylate-based resins. These resins are all compatible with the Dbs/methylation chemistry - alkylation, reductive alkylation, and mild acidolysis - because these reactions are performed under mild conditions compatible with the aforementioned resin types, since these resins are made of ethers, esters, amides, and polymeric backbones inert to the reaction conditions described herein. "Resin" includes, polystyrene resins such as PS-DVB resin, PEG-based resins such as CLEAR resin, ChemMatrix resin, and PEGA resin, 'hybrid' resins - part PEG and part polystyrene - such as TentaGel and HypoGel, and silica/methacrylate resins such as SynBeads and Functionalized Silica.

The term "linker" includes, any chemical entity that connects the amino acid or peptide to the resin, such as the Rink Amide Linker - that is stable for short periods of low concentrations of TFA, benzyl ester linkage (Merrifield Resin), 4-hydroxymethylphenylacetic acid linkage (PAM Linker), p-Alkoxybenzyl ester linkage (Wang Resin), 4-hydroxymethylphenoxyacetic acid linkage (Sheppard Linker), 4-methylbenzhydrylamide linkage (MBHA Resin), Rink Amide Resin, Rink Amide Linker, and 4-(4-aminomethyl,3,5-dimethoxyphenoxy)butyric acid linker (PAL Linker).

The phrase "coupled to a solid phase resin," as used herein, means connected or bound to a solid support or resin via a linker. For example, an amino acid coupled to a solid phase resin includes, an amino acid, such as Arg(Pbf), coupled to PS-DVB resin with a Rink Amide linker.

Technical and scientific terms used herein have the meaning commonly understood by one of skill in the art to which the present invention pertains, unless otherwise defined. Reference is made herein to various methodologies and materials known to those of skill in the art. Standard reference works setting forth the general principles of pharmacology include Goodman and Gilman's The Pharmacological Basis of Therapeutics, 10th Ed., McGraw Hill Companies Inc., New York (2001). Any suitable materials and/or methods known to those of skill can be utilized in carrying out the present invention. However, preferred materials and methods are described. Materials, reagents and the like to which reference are made in the following description and examples are obtainable from commercial sources, unless otherwise noted.

A bond drawn into ring system (as opposed to connected at a distinct vertex) indicates that the bond may be attached to any of the suitable ring atoms.

The term "alkyl" as used herein denotes an unbranched or branched chain, saturated, monovalent hydrocarbon residue containing 1 to 10 carbon atoms. The term "lower alkyl" denotes a straight or branched chain hydrocarbon residue containing 1 to 6 carbon atoms. "C1-10 alkyl" as used herein refers to an alkyl composed of 1 to 10 carbons. Examples of alkyl groups include, lower alkyl groups including methyl, ethyl, propyl, i-propyl, n-butyl, i-butyl, t-butyl or pentyl, isopentyl, neopentyl, hexyl, heptyl, and octyl.

When the term "alkyl" is used as a suffix following another term, as in "haloalkyl," this is intended to refer to an alkyl group, as defined above, being substituted with one to three substituents selected from the other specifically-named group. Thus, for example, "haloalkyl" denotes the radical R'R" , wherein R' is a halogen radical, and R" is an alkylene radical as defined herein with the understanding that the attachment point of the halolalkyl moiety will be on the alkylene radical.

The term "alkylene" or "alkylenyl" as used herein denotes a divalent saturated linear hydrocarbon radical of 1 to 10 carbon atoms (e.g., (CH₂)n)or a branched saturated divalent hydrocarbon radical of 2 to 10 carbon atoms (e.g., -CHMe- or -CH₂CH(i-Pr)CH₂-), unless otherwise indicated. Except in the case of methylene, the open valences of an alkylene group are not attached to the same atom. Examples of alkylene radicals include, methylene, ethylene, propylene, 2-methyl-propylene, 1,1-dimethyl-ethylene, butylene, 2-ethylbutylene.

The abbreviation "Dbs-Cl," as used herein refers to 5-chlorodibenzosuberane.

The abbreviation "Dbs," as used herein, refers to dibenzosuberane

The abbreviation "TFA," as used herein, refers to trifluoroacetic acid.

The abbreviation "Pbf," as used herein, refers to 2,2,4,6,7-pentamethyldihydrobenzofuran-5-sulfonyl.

Commonly used abbreviations include: acetyl (Ac), azo-bis-isobutyrylnitrile (AIBN), atmospheres (Atm), 9-borabicyclo[3.3.1]nonane (9-BBN or BBN), tert-butoxycarbonyl (Boc), di-tert-butyl pyrocarbonate or boc anhydride (BOC₂O), benzyl (Bn), butyl (Bu), Chemical Abstracts Registration Number (CASRN), benzyloxycarbonyl (CBZ or Z), carbonyl diimidazole (CDI), 1,4-diazabicyclo[2.2.2]octane (DABCO), diethylaminosulfur trifluoride (DAST), dibenzylideneacetone (dba), 1,5-diazabicyclo[4.3.0]non-5-ene (DBN), 1,8-diazabicyclo[5.4.0]undec-7-ene (DBU), N,N'-dicyclohexylcarbodiimide (DCC), 1,2-dichloroethane (DCE), dichloromethane (DCM), diethyl azodicarboxylate (DEAD), di-iso-propylazodicarboxylate (DIAD), di-iso-butylaluminumhydride (DIBAL or DIBAL-H), di-iso-propylethylamine (DIPEA), N,N-dimethyl acetamide (DMA), 4-N,N-dimethylaminopyridine (DMAP), N,N-dimethylformamide (DMF), dimethyl sulfoxide (DMSO), 1,1'-bis-(diphenylphosphino)ethane (dppe), 1,1'-bis-(diphenylphosphino)ferrocene (dppf), 1-(3-dimethylaminopropyl)-3-ethylcarbodiimide hydrochloride (EDCI), ethyl (Et), ethyl acetate (EtOAc), ethanol (EtOH), 2-ethoxy-2H-quinoline-1-carboxylic acid ethyl ester (EEDQ), diethyl ether (Et20), O-(7-azabenzotriazole-1-yl)-N, N,N'N'-tetramethyluronium hexafluorophosphate acetic acid (HATU), acetic acid (HOAc), 1-N-hydroxybenzotriazole (HOBt), high pressure liquid chromatography (HPLC), iso-propanol (IPA), lithium hexamethyl disilazane (LiHMDS), methanol (MeOH), melting point (mp), MeS02- (mesyl or Ms), methyl (Me), acetonitrile (MeCN), m-chloroperbenzoic acid (MCPBA), mass spectrum (ms), methyl t-butyl ether (MTBE), N-bromosuccinimide (NBS), N-carboxyanhydride (NCA), N-chlorosuccinimide (NCS), N-methylmorpholine (NMM), N-methylpyrrolidone (NMP), pyridinium chlorochromate (PCC), pyridinium dichromate (PDC), phenyl (Ph), propyl (Pr), iso-propyl (i-Pr), pounds per square inch (psi), pyridine (pyr), room temperature (rt or RT), tert-butyldimethylsilyl or t-BuMe2Si (TBDMS), triethylamine (TEA or Et3N), 2,2,6,6-tetramethylpiperidine 1-oxyl (TEMPO), triflate or CF3SO2- (Tf), 1,1'-bis-2,2,6,6-tetramethylheptane-2,6-dione (TMHD), O-benzotriazol-1-yl-N,N,N',N'-tetramethyluronium tetrafluoroborate (TBTU), thin layer chromatography (TLC), tetrahydrofuran (THF), trimethylsilyl or Me3Si (TMS), p-toluenesulfonic acid monohydrate (TsOH or pTsOH), 4-Me-C6H4SO2- or tosyl (Ts), N-urethane-N-carboxyanhydride (UNCA).

Conventional nomenclature including the prefixes normal (n), iso (i-), secondary (sec-), tertiary (tert-) and neo have their customary meaning when used with an alkyl moiety. (J. Rigaudy and D. P. Klesney, Nomenclature in Organic Chemistry, IUPAC 1979 Pergamon Press, Oxford.).

In general, the nomenclature used in this Application is based on AUTONOMTM v.4.0, a Beilstein Institute computerized system for the generation of IUPAC systematic nomenclature. If there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

In the development of a method for ^{α}*N*-methylation chemistry suitable for on-resin methylation compatible with Fmoc/tBu SPPS and with amino acids bearing protected or unprotected nucleophilic side-chains, notably Arg(Pbf), Met, Cys, and Trp, it was opted not to use Fukuyama's 2,4-dinitrobenzenesulfonamide owing to the hazards (explosivity of azodicarboxylate reagents) and heterogeneity issues (precipitation of triphenylphosphine oxide) associated with Mitsunobu reactions in solid phase chemistry. Furthermore, as expected, Fukuyama's 2-nitrobenzenesulfonamide chemistry proved intractable; while some desired product was obtained, this chemistry was low-yielding and certainly not scaleable. Efforts turned to the original Undén chemistry, which is completely compatible with tosylamide-based Arg side-chain protection commonly employed in Boc/Bzl SPPS. However, the relatively harsh acidolytic deprotection conditions needed for post-methylation ^{α}*N*-Dod deprotection are incompatible with the side-chain protection and peptide-resin anchorage used in the Fmoc/tBu chemistry. It was therefore intended to identify an alternative transient ^{α}*N-*protecting group that could be removed under milder conditions (≤10 vol% TFA in CH₂Cl₂ for ca. 5 min), which would preserve the integrity of protecting groups, such as Arg(Pbf) and Tyr(t-Bu), as well as linkers, such as Rink Amide linker, which is cleaved under prolonged acid treatment at higher concentrations of TFA. N-trityl amines are deprotected under extremely mild conditions (1% TFA, v/v in CH₂Cl₂). However, it was found that - in agreement with Unden's observations - ^{α}*N*-tritylation effectively shields the α-amino group from reductive alkylation, presumably due to the extreme steric hindrance from the bulky trityl skeleton.

A transient ^{α}*N*-protecting protecting group with a high degree of acid lability required for Fmoc/tBu chemistry and less steric hindrance around the α-amino group was required. Of the many protecting groups and linkers used in organic chemistry, one acid labile linkage agent, Ramage's tricyclic amide linker, is based on the dibenzosuberane (Dbs) skeleton (Ramage, R.; Irving, S. L.; McInnes, C. Tetrahedron Lett. 1993, 34, 6599-6602). The ^{α}*N*-dibenzosuberyl amine is considerably less sterically crowded than an ^{α}*N*-trityl amine, as the α-carbon is tertiary in the former vs. quaternary in the latter, and is therefore more accessible for reductive alkylation, on a par with the Dod group. Although Dbs has been previously reported as an amine-protecting group due to its lability to acidolysis and catalytic hydrogenation (Pless, J. Helv. Chim. Acta 1976, 59, 499-512, Hong, C. Y.; Overman, L. E.; Romero, A. Tetrahedron Lett. 1997, 38, 8439-8442), it was previously known to be labile to ca. 10-20% TFA (v/v in CH₂Cl₂), which is too high for the present application but acceptable for its usual application as a peptide-resin linkage agent cleavable in a global deprotection step. Use of this protecting group as a transient ^{α}*N-*protecting group, as opposed to the standard secondary amide nitrogen protecting group, as well as the corresponding minimum acid lability of this protecting group has not been reported previously and is described herein.

As described in further detail below, the Dbs group has successfully been employed as a transient ^{α}*N-*protecting group, as, it is smoothly deprotected by dilute TFA, which is compatible with Fmoc/tBu chemistry, side-chain protecting groups, and the peptide-resin anchorage. The three-step methylation sequence - protection, methylation, and deprotection - was performed using the Dbs group (General Scheme). In Example 1, the peptide-resin after Arg(Pbf) Fmoc deprotection was easily N-alkylated with Dbs-Cl in the presence of DIEA in NMP.

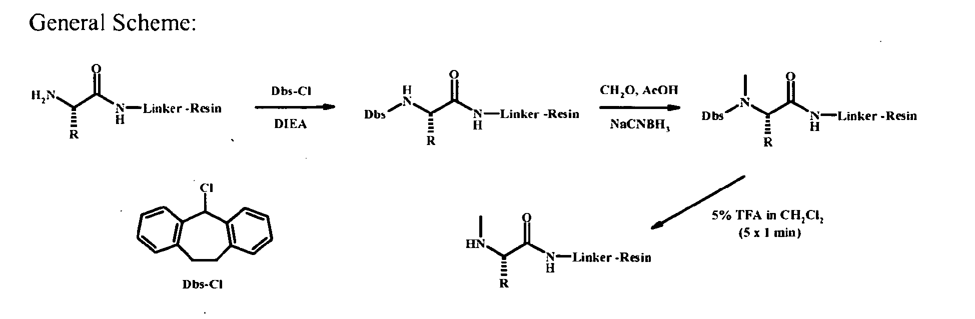

This reaction was easily monitored by the ninhydrin test and was found to be complete ter overnight reaction (and later demonstrated to be complete in 6 hours). The reductive methylation step was modeled based on Undén's original reaction conditions, with the addition of THF as a cosolvent to coordinate borate salts and prevent their precipitation in the resin interior, as well as with a reduced water content to improve resin swelling. A slightly altered stoichiometry was also employed to maximize methylation yield, but nonetheless the methylation step was repeated to ensure quantitative methylation. The Dbs group was then removed with 5 vol% TFA in CH₂Cl₂ (5 x 1 min), after which the resin was free-based with DIEA and then assayed by ninhydrin. Although both primary and secondary amines react with ninhdyrin, only primary amines allow for the formation of the soluble Ruhemann's purple adduct; secondary amines take on a faint reddish color on the resin. The absence of the Ruhemann's purple adduct therefore provided confirmation that the methylation reaction proceeded quantitatively.

After the on-resin methylation step, a Gln residue was coupled and a sample of the resin-bound Fmoc-QmRY-CONH₂ was cleaved and assayed by RP-HPLC. This material was found to be of comparable purity to the purified tripeptide obtained using commercially available Fmoc-MeArg(Mtr)-OH, suggesting that methylation was indeed quantitative, and reductive alkylation conditions did not contaminate the peptide-resin with precipitated salts which would have impeded the subsequent coupling. Chain assembly was then completed to the N-terminus, and the purity of the peptide was compared using both the on-resin Dbs methylation route described herein and an alternative route using Fmoc-MeArg(Mtr)-OH. After cleavage, purification, and lyophilization, the crude material from the Dbs/on-resin methylation route was significantly more pure and the isolated yield was nearly three times higher.

Peptide syntheses employing Fmoc-MeArg(Mtr)-OH often afford complex mixtures, difficult chromatography, and low isolated yields, particularly when the target sequence contains Trp, Met, or unnatural amino acid residues. These problems can often be ascribed to the use of the Mtr group for Arg protection. Because the use of the more acid labile Arg(Pbf) is not associated with these complications, this Dbs on-resin N-methylation methodology facilitates peptide build scaleup using the inexpensive and widely available Fmoc-Arg(Pbf)-OH. Moreover, the chemistry is by far the most generally applicable chemistry for on-resin methylation in the context of SPPS. Similarly, in Boc/Bzl chemistry, the non-commercially available Dod-Cl reagent can be replaced with the commercially available Dbs-Cl. In the context of Fmoc/tBu chemistry, this chemistry is the first to be demonstrated to be completely compatible with Arg(Pbf), and is also compatible and effective for ^{α}*N*-methylation of other amino acids, including Met, Cys, and Trp. However, Dbs/on-resin methylation chemistry effectively results in side-chain methylation of His(Trt) residues, which is not unexpected, as Kaljuste and Undén report the same observation using His(Dnp) in the context of Boc/Bzl SPPS. This problem may similarly be circumvented by foregoing side-chain protection on the His side chain, a tactic which could also plausibly be employed in Fmoc/tBu SPPS through the use of a number of specialized His side chain protection strategies.

### Examples

### Materials

NaCNBH₃ (Aldrich cat# 156159), formaldehyde, 37 wt% (12.3 M) in water (Aldrich cat# 252549), 5-chlorodibenzosuberane (Dbs-Cl) (Aldrich cat# C34308). All operations are performed at ambient temperature, pressure, and atmosphere. All washes are 200 mL, with a 1 min stirring period. All percentages are volume %. The base resin used is Polymer Labs PL-AMS resin, 1.0 mmol/g.

### General Procedure

The following general procedure applies to the ^{α}*N-*methylation of any amino acid except Histidine, which undergoes excess methylation onto one of the imidazole side chain nitrogen atoms. The amino acid or peptide can be linked by any linker, as defined herein, to any resin, as defined herein, for ^{α}*N-*methylation of the terminal amino acid according to the following 3 general steps:

### Step 1 - Dbs Protection:

After Fmoc deprotection, 1 equivalent of an amino acid or peptide on resin is washed appropriately with DMF and drained. 5 equivalents of Dbs-Cl is dissolved in sufficient DMF to allow for resin swelling, to which 25 equivalents DIEA are added, and the solution is then added to the resin, which is then stirred for 4 hours. Ninhydrin is negative at this point. The resin is then washed (5x DMF) and left suspended in DMF until the next step.

### Step 2 - Methylation:

The Methylation Reagent Solution is prepared immediately before use as 25 equivalents formaldehyde in approximately 3:1:0.05 NMP/THF/AcOH.

The resin from Step 1 is drained and the Methylation Reagent Solution is added. The resin is then stirred for several minutes to preform the methyl imine. 10 equivalents of NaCNBH₃ is then added to the stirring peptide resin via powder funnel, and stirred for several hours, after which it is drained, washed with DMF, and left suspended in DMF until the next step.

### Step 3 - Dbs Deprotection:

The resin from Step 2 is drained and washed with CH₂Cl₂ to remove traces of DMF from the resin, and drained again. The resin is then treated in a batchwise fashion with 5% TFA in CH₂Cl₂ with stirring, washed with CH₂Cl₂ and DMF, and then is available for subsequent coupling via DIC/HOAt chemistry.

### Example 1

### Step 1 - Dbs Protection:

After Fmoc deprotection to yield 0.01 mol H₂N-Arg(Pbf)-Tyr(tBu)-Rink-Ahx-Resin, the resin was washed appropriately (2x DMF, 2x CH₂Cl₂, 2x DMF) and drained. 0.05 mol Dbs-Cl was dissolved in 150 mL DMF, to which 50 mL 1:1 DIEA:Toluene was added. This solution was then added to the resin, which was then stirred for 4 hours. Ninhydrin is negative at this point. The resin was then washed (5x DMF) and left suspended in DMF until the next step.

### Step 2 - Methylation:

The following Methylation Reagent Solution was prepared immediately before use: 300 mL NMP, 80 mL THF, 20 mL Formaldehyde solution (0.246 mol), 4 mL Acetic acid.

The resin from Step 1 was drained, after which athe Methylation Reagent Solution was added. The resin was then stirred 15 mins to preform the methyl imine. 0.1 mol (6.2 g) NaCNBH₃ was then weighed out and added in one portion to the stirring peptide resin via powder funnel, with a minimal (10-20 mL) chase of NMP as necessary. The resin was stirred for 6 hrs, after which it was drained, washed (5x DMF), drained again, and the entire methylation procedure repeated with a fresh cocktail, this time overnight (∼18 hours). The resin was then drained, washed (5x DMF), and left suspended in DMF until the next step.

### Step 3 - Dbs Deprotection:

The resin from Step 2 was drained and washed (5x CH₂Cl₂) to remove traces of DMF from the resin, then drained again. The resin was then treated in a batchwise fashion with 5% TFA in CH₂Cl₂ (5 x 1 min) with stirring. The resin was then washed (2x CH₂Cl₂, 2x DMF), and then subjected to the subsequent coupling via DIC/HOAt chemistry using an automated cycle which commences with an Fmoc deprotection, which is unnecessary in this case, as the piperidine treatment usually serves to liberate the N-terminus as a free base.

## Claims

1. A method for ^{α}*N*-methylating of an amino acid or a peptide on a solid phase resin comprising the steps of:
a) contacting the amino acid or peptide with 5-chlorodibenzosuberane (Dbs-Cl),
b) contacting the product of step a) with formaldehyde or a protected formaldehyde equivalent,
c) contacting the product of step b) with a reducing agent; and
d) contacting the product of step c) with 5% TFA.

2. The method of claim 1, wherein the amino acid is selected from the group of Arg (Pbf) [Pbf = 2,2,4,6,7-pentamethyldihydrobenzofuran-5-sulfonamide], methionine, tryptophan, cysteine.

3. The method of claim 1 or 2, wherein the amino acid comprises a side-chain protecting group.

4. The method of claims 1 to 3, wherein the reducing agent is NaCNBH₃ or NaBH(OAC)₃.

## Patentansprüche

1. Verfahren zur ^{α}*N*-Methylierung einer Aminosäure oder eines Peptids an einem Festphasenharz, umfassend die Schritte:
a) Kontaktieren der Aminosäure oder des Pepids mit 5-Chlordibenzosuberan (Dbs-Cl),
b) Kontaktieren des Produktes von Schritt a) mit Formaldehyd oder einem geschützten Formaldehydäquivalent,
c) Kontaktieren des Produktes von Schritt b) mit einem Reduktionsmittel und
d) Kontaktieren des Produktes von Schritt c) mit 5%iger TFA.

2. Verfahren nach Anspruch 1, wobei die Aminosäure aus der Gruppe Arg(Pbf) [Pbf = 2,2,4,6,7-Pentamethyldihydrobenzofuran-5-sulfonamidl, Methionin, Tryptophan, Cystein ausgewählt ist.

3. Verfahren nach Anspruch 1 oder 2, wobei die Aminosäure eine Seitenkettenschutzgruppe umfasst.

4. Verfahren nach den Ansprüchen 1 bis 3, wobei das Reduktionsmittel NaCNBH₃ oder NaBH(OAc)₃ ist.

## Revendications

1. Procédé d'^{α}N-méthylation d'un aminoacide ou d'un peptide sur une résine en phase solide, comprenant les étapes de:
a) mise en contact de l'aminoacide ou du peptide avec du 5-chlorodibenzosubérane (Dbs-Cl),
b) mise en contact du produit de l'étape a) avec du formaldéhyde ou un équivalent de formaldéhyde protégé,
c) mise en contact du produit de l'étape b) avec un agent réducteur, et
d) mise en contact du produit de l'étape c) avec du TFA à 5 %.

2. Procédé selon la revendication 1, dans lequel l'aminoacide est choisi dans le groupe de Arg (Pbf) [Pbf = 2,2,4,6,7-pentaméthyldihydrobonzofurane-5-sulfonamide], de la méthionine, du tryptophane, de la cystéine.

3. Procédé selon la revendication 1 ou 2, dans lequel l'aminoacide comprend un groupe protecteur de la chaîne latérale.

4. Procédé selon les revendications 1 à 3, dans lequel l'agent réducteur est NaCNBH₃ ou NaBH(OAc)₃.
